# EUROPEAN PATENT APPLICATION

(11) **EP 2 160 975 A1**
(43) Date of publication of application: **10.03.2010**
(21) Application number: 09169764.9
(22) Date of filing: 08.09.2009
(51) Int. Cl.: A61B 5/00, A61B 5/02, G06F 19/00

(54) **Device for controlling a safety signal range of a medical patient monitoring apparatus**

(30) Priority: 08.09.2008 EP 08015792
(71) Applicant: Saad Specialist Hospital Co., Al-Khobar 31952 (SA)
(72) Inventor: Holzhausen, Rudolf Johannes, 31952, Al-Khobar (SA)
(74) Representative: Land, Addick Adrianus Gosling

(57) **Abstract**

The invention relates to a device for controlling a safety signal range of a medical patient monitoring apparatus. Such a medical patient monitoring apparatus is for instance an Electrocardiograph in emergency and intensive care areas in hospitals. The invention provides a device for controlling a safety signal range of a medical patient monitoring apparatus according to the invention comprising data acquisition means capable of acquiring data representing a patient monitoring parameter, processing means for processing the acquired data for providing a reference safety signal range for said patient monitoring parameter, and adjusting means for adjusting the safety signal range on the basis of reference safety signal range.

## Description

The invention relates to a device for controlling a safety signal range of a medical patient monitoring apparatus. Such a medical patient monitoring apparatus is for instance an Electrocardiograph in emergency and intensive care areas in hospitals.

Known medical patient monitoring apparatus have the ability to adjust pre-set safety signal ranges on medical patient monitoring devices. As standard medical practice these pre-set safety signal ranges are often expanded during the course of a clinical procedure, such as patient physical examination, patient hygiene care, administration of medications and such standard clinical treatments and procedures.

A drawback of the known medical patient monitoring apparatus is that in busy and demanding health care situations there exists the possibility of clinical staff failing to return safety signal ranges to accepted normal levels after completion of clinical procedures, which themselves are liable to trigger safety signals falsely.

The invention has for its object to obviate or at least alleviate this drawback.

For this purpose the invention provides a device for controlling a safety signal range of a medical patient monitoring apparatus according to the invention comprising data acquisition means capable of acquiring data representing a patient monitoring parameter, processing means for processing the acquired data for providing a reference safety signal range for said patient monitoring parameter, and adjusting means for adjusting the safety signal range on the basis of reference safety signal range.

This device according to the invention makes it possible to provide a supervisory capability to the effective and safe use of the ability for the clinical staff to adjust safety signal ranges on medical patient monitoring devices. This has the advantage for instance that chance of human error by way of the user not returning the limits of the safety signal range to their clinically accepted upper and lower value, is reduced.

In a preferred embodiment of the device according to the invention said adjusting means is designed to adjust the safety signal range after a predetermined time interval.

In a further embodiment of the device according to the invention said adjusting means is designed to adjust the safety signal range in creeping stages.

In a further embodiment of the device according to the invention said data representing a patient monitoring parameter is real-time data and said processing means processes said real-time data in real-time.

In a further embodiment of the apparatus according to the invention said processing means is designed to process said acquired data by retrieving from a database the corresponding reference safety signal range.

In a further embodiment of the apparatus according to the invention said processing means is designed to process said acquired data by means of mathematical algorithms, preferably predictive and dynamic assessment algorithms. This approach eliminates the need for comparative analysis and/or historically based evaluation methods using for instance databases.

The present invention will be further elucidated herein below on the basis of an exemplary embodiment which is shown schematically in the accompanying figure. This is a non-limitative exemplary embodiment.

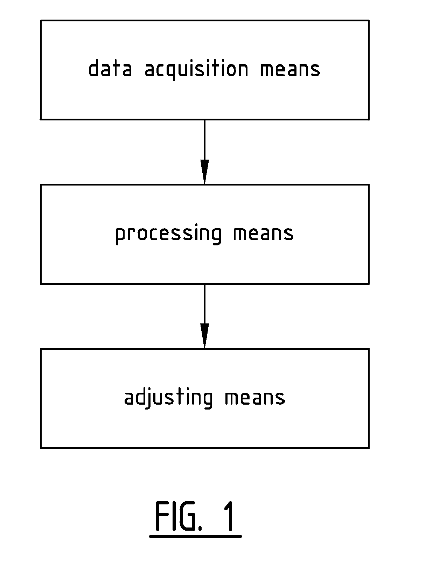

Figure 1 shows schematically the device for controlling a safety signal range of a medical patient monitoring apparatus according to the invention comprising data acquisition means capable of acquiring data representing a patient monitoring parameter, processing means for processing the acquired data for providing a reference safety signal range for said patient monitoring parameter, and adjusting means for adjusting the safety signal range on the basis of reference safety signal range.

This device automatically adjusts upper and lower alarm limits of the safety signal range set by clinical staff. This device ensures that settings made will automatically correct themselves if the care provider does not constantly monitor the original limits set. This further ensures that these settings are not permanent, rather only used as a temporary adjusted scale to allow a clinical procedure to be undertaken, which would normally trigger the alarm, if the settings remained in their normal mode. In essence this component ensures that the parameters monitoring the patient are set within accepted clinical limits.

The device continuously monitors the data stream of live vital signs coming from the patient via the patient monitoring equipment, such as patient monitors, ventilators and anesthesia devices. This device reviews these outputs and live vital sign parameters whilst simultaneously monitoring the pre-set upper and lower alarm limits of these parameters, if the component identifies an irregularity in the settings it automatically, after a prescribed time interval, over-rides the settings controls on the patient monitoring device and ensures that limits in creeping stages return to clinically accepted and safe levels.

During real-time monitoring the data is continually analyzed by software driven mathematical algorithms that continuously monitor and automatically over-ride set upper and lower limits, after a pre-set time interval. Once activated this device incrementally adjusts the upper and lower alarm levels to the clinically accepted or pre-set range. The user has the additional option to enable or disable this device.

## Claims

1. Device for controlling a safety signal range of a medical patient monitoring apparatus, comprising:
data acquisition means capable of acquiring data representing a patient monitoring parameter;
processing means for processing the acquired data for providing a reference safety signal range for said patient monitoring parameter;
adjusting means for adjusting the safety signal range on the basis of reference safety signal range.

2. Device as claimed in claim 1, wherein said adjusting means is designed to adjust the safety signal range after a predetermined time interval.

3. Device as claimed in any of the forgoing claims, wherein said adjusting means is designed to adjust the safety signal range in creeping stages.

4. Device as claimed in any of the forgoing claims, wherein said data representing a patient monitoring parameter is real-time data and said processing means processes said real-time data in real-time.

5. Device as claimed in any of the forgoing claims, wherein said processing means is designed to process said acquired data by retrieving from a database the corresponding reference safety range.

6. Device as claimed in any of the forgoing claims, wherein said processing means is designed to process said acquired data by means of mathematical algorithms, preferably predictive and dynamic assessment algorithms.

7. Method for controlling a safety signal range of a medical patient monitoring apparatus comprising the use of a device as claimed in any of the foregoing claims.
